# EUROPEAN PATENT SPECIFICATION

(11) **EP 1 615 608 B1**
(45) Date of publication and mention of the grant of the patent: **28.02.2007**
(21) Application number: 04741467.7
(22) Date of filing: 16.04.2004
(51) Int. Cl.: A61H 33/06, A47K 3/28, A47K 3/40

(54) **STALL EQUIPPED WITH A STEAM GENERATOR**
DUSCHKABINE MIT DAMPFERZEUGER
CABINE EQUIPEE D'UN GENERATEUR DE VAPEUR

(30) Priority: 18.04.2003 IT BO20030236
(43) Date of publication of application: 18.01.2006
(73) Proprietor: TEUCO GUZZINI S.p.A., 62010 Montelupone (IT)
(72) Inventor: GUZZINI, Mauro, I-62019 Recanati (IT)
(74) Representative: Jorio, Paolo
(86) International application number: PCT/EP2004/050550
(87) International publication number: WO 2004/091470

(56) References cited:
- EP-A- 1 210 897
- EP-A- 1 212 970
- EP-A- 1 250 876
- WO-A-93/09752
- DE-A- 1 566 484
- US-A- 4 432 103

## Description

### TECHNICAL FIELD

The present invention relates to a stall equipped with a steam generator.

### BACKGROUND ART

As is known, stalls equipped with a steam generator currently comprise a base body; a shower stand fixed to the base body; a structure defined by lateral walls, one of which for access, and by a top wall; and at least one steam outlet.

A first type of currently marketed stall has the generator installed at the top. Stalls of this type are complicated and therefore expensive to produce, on account of installation of the generator at the top of the stall requiring a supporting structure of given mechanical characteristics capable of supporting the generator. Also, steam channels from the generator to the outlet must be formed in the structure.

In a second type of stall, described and illustrated in US Patent 5,416,931, the steam generator is installed in a gap formed in the base body, beneath the shower stand which defines a panel protecting the generator. Access to the gap is difficult, by requiring removal of the shower stand, which, in addition to the steam nozzles, also comprises a water drain fitting. This therefore complicates maintenance of the stall, which, as is known, is normally carried out regularly.

Another important point to note is that, in both the above types of stall, the nozzles, being located on the shower stand, are subject to clogging by hair and other falling matter, and must therefore be cleaned frequently, which can only be done easily by first removing the shower stand from the base body. Moreover, the nozzles being installed in the normal user tread area, the steam jets are directed onto the user's soles which, as is known, are the most heat-sensitive part of the human body.

Another type of shower stall is disclosed in DE-A-1 566 484 or US-A-4 432 103.

### DISCLOSURE OF INVENTION

It is an object of the present invention to provide a stall equipped with a steam generator and designed to eliminate the aforementioned drawbacks.

According to the present invention, there is provided a stall, as claimed in Claim 1.

### BRIEF DESCRIPTION OF THE DRAWINGS

A non-limiting embodiment of the present invention will be described by way of example with reference to the accompanying drawings, in which:
Figure 1 shows a view in perspective of a stall in accordance with the teachings of the present invention;
Figure 2 shows a larger-scale section of a bottom portion of the Figure 1 stall;
Figure 3 shows a plan view of the Figure 2 portion with parts removed for clarity.

### BEST MODE FOR CARRYING OUT THE INVENTION

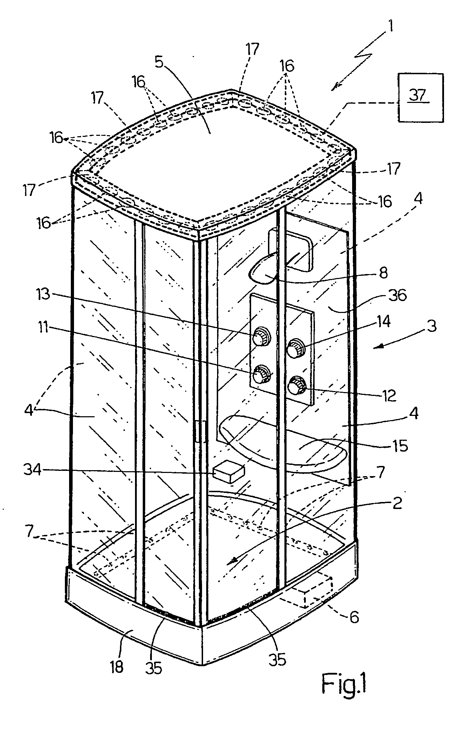

Number 1 in Figure 1 indicates as a whole a stall comprising a shower stand 2; a prismatic structure 3 defined by four lateral walls 4, at least one of which for access, and by a top wall 5; a steam generator 6; and a number of nozzles 7 for directing steam into stall 1.

Stall 1 also comprises a shower head 8 (known); a device 11 (known) for adjusting water outflow from shower head 8; a mixing device 12 (known) for adjusting the temperature of the water from shower head 8; a device 13 for activating steam supply from nozzles 7; and a device 14 for adjusting the temperature inside stall 1.

Stall 1 also comprises a seat 15; and a number of lamps 16 fitted to the top of stall 1 by means of a supporting strip 17. Lamps 16 are equally spaced along the peripheral edge of top wall 5, direct light downwards onto lateral walls 4, and emit coloured-light beams (even of different colours from one lamp to another) so as to define a chromotherapy device.

In an embodiment not shown, lamps 16 may be replaced with a single central lamp for chromotherapy treatment.

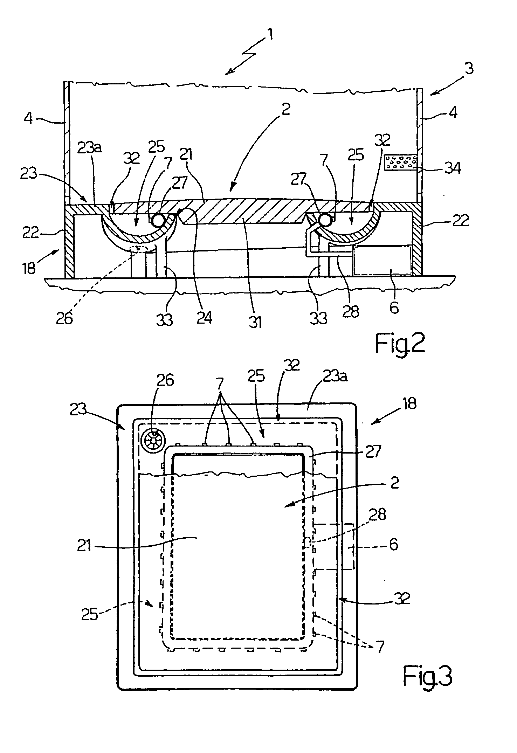

With reference to Figures 2 and 3, shower stand 2 comprises a base body 18 preferably molded in one piece from plastic material; and a plate 21 also molded from plastic material, and fitted removably to base body 18. The top surface of plate 21 is the user tread area. Base body 18 is substantially box-shaped with a rectangular outer perimeter. More specifically, base body 18 comprises four lateral walls 22; and a substantially horizontal top wall 23 having a rectangular, central, vertical through hole 24. Top wall 23 is defined by a peripheral portion 23a lying in a horizontal plane and projecting from the top edge of lateral walls 22; and by a portion defined by a depression 25 and located between portion 23a and the peripheral edge of hole 24.

With reference to Figures 2 and 3, depression 25 surrounds hole 24 and, in plan view, has a rectangular perimeter. A drain 26 is located at one corner of the rectangular perimeter of depression 25, and is connected by a known siphon (not shown) to a known drain conduit (not shown). The bottom wall of depression 25 rises gradually from the corner of its perimeter where drain 26 is formed, to the diagonally opposite corner, so that depression 25 defines a channel towards drain 26.

With reference to Figures 2 and 3, a rectangular conduit 27 is located inside depression 25, is connected by a pipe 28 to steam generator 6, and has a number of nozzles 7 equally spaced along the longitudinal axis of conduit 27 and facing portion 23a. Plate 21 has a bottom face, in the central portion of which is formed a rectangular projection 31 which, in use, engages hole 24. Plate 21 has a rectangular perimeter sized to define, between the perimeter of plate 21 and the inner peripheral edge of portion 23a, an opening 32 which, in plan view, is obviously rectangular. Finally, base body 18 is formed in one piece with a number of uprights 33 which rest on the floor at the bottom and support depression 25 at the top.

With reference to Figures 2 and 3, steam generator 6 is housed in the gap defined in base body 18 between wall 23 and the floor, and which is easily accessible by lifting plate 21. In addition to chromotherapy, stall 1 may also be equipped for aromatherapy by aromas placed in depression 25 or inside a container 34 fixed to any one of lateral walls 4 of structure 3. Plate 21 (Figure 2) has a top face shaped to define not only a non-slip tread surface but also channels for channelling water through opening 32 into depression 25.

With reference to Figure 1, walls 4 and 5 are preferably transparent and made of plastic material or glass. Walls 4 are supported on base body 18, at portion 23a, so that opening 32 is close to the bottom edge of walls 4. Access to stall 1 may be through a door or, as shown in Figure 1, through walls 4 sliding along guides 35 on base body 18. Obviously, one or two of walls 4 may be defined by the masonry wall/s of the room in which stall 1 is installed.

With reference to Figure 1, stall 1 may be fitted inside with an equipment panel 36 supporting shower head 8 and devices 11, 12, 13, 14. Equipment panel 36 naturally has all the channelling necessary for operation of said devices, and may be formed in one piece in known manner with seat 15. Stall 1 is provided, outside structure 3, with an electric power supply 37; and equipment panel 36 may comprise known nozzles (not shown) for directing water jets onto the user's body; in which case, a known selecting device (not shown) is required. Equipment panel 36 may also comprise nozzles (not shown) for emitting lateral steam jets; in which case, a selecting device (not shown) is also required.

In actual use, the steam from nozzles 7 issues from depression 25 through opening 32 located, as stated, close to walls 4 of structure 3, thus forming four rising columns of steam close to walls 4 and surrounding the body of the user - not shown, but standing on plate 21 - on all four sides. At dew point, the steam condenses on walls 4 and the user's body, and the condensation flows through opening 32 into depression 25 where it is channelled to drain 26.

The many advantages of the present invention will be clear from the foregoing description.

In particular, stall 1 may be used both as a shower and a steam bath, and is straightforward in design and quick and easy to assemble. Structure 3 is made of steam-resistant material, and, not being required to support heavy weights, is simpler and lighter. All of which factors greatly reduce the production cost of stall 1 as compared with known stalls.

Stall 1 is also equipped for chromotherapy and aromatherapy. What is more, chromotherapy is enhanced by the formation of four rising steam columns, and by using different-coloured lights, and transparent lateral walls 4 of structure 3. Moreover, plate 21 provides for shielding the steam jets from nozzles 7, which are directed towards walls 4, as opposed to directly onto the highly sensitive soles of the user.

Obviously, one of the main advantages of stall 1 lies in easy access to steam generator 6 and nozzles 7, thus greatly simplifying maintenance, Moreover, nozzles 7 are safeguarded against clogging by hair or other falling matter on plate 21.

Finally, depression 25 provides simultaneously for channelling the steam upwards and the condensation and water to the drain.

Clearly, changes may be made to stall 1 as described and illustrated herein without, however, departing from the scope of the present invention.

In particular, shower stand 2 and structure 3, and consequently also depression 25 and opening 32, may be circular.

In a further embodiment not shown, to prevent load losses in conduit 27, each nozzle 7 is connected directly to the steam generator by a relative conduit (not shown); in which case, stall 1 has no conduit 27.

## Claims

1. A stall (1) comprising a shower stand (2); a box structure (3) defined by lateral walls (4), in which an access is formed, and by a top wall (5); a steam generator (6); and nozzles (7) located on the shower stand (2) to direct steam inside the stall (1);
the shower stand comprising a base body (18);
the stall (1) **characterized in that** said shower stand (2) comprises also a tread plate (21) for diverting the steam from said nozzles (7) towards said lateral walls (4); **in that** between said base body (18) and said tread plate (21) being defined an opening (32) close to said lateral walls (4); and **in that** said nozzles (7) are installed beneath said tread plate (21) so that the steam rises through said opening (32).

2. A stall (1) as claimed in Claim 1, **characterized in that** the plate (21) is fitted removably to said base body (18).

3. A stall (1) as claimed in Claim 2, **characterized in that** said base body (18) is box-shaped, and comprises a top wall (23) having a central through hole (24); and **in that** said steam generator (6) is housed inside the base body (18); said hole (24) defining the access to the space enclosed by said base body (18).

4. A stall (1) as claimed in Claim 3, **characterized in that** said plate (21) comprises, on its bottom face, a projection (31) which engages said hole (24).

5. A stall (1) as claimed in Claim 3 and/or 4, **characterized in that** said top wall (23) of said base body (18) comprises a depression (25) surrounding said hole (24) and housing said nozzles (7); said depression (25) channelling the steam from said nozzles (7) to said opening (32).

6. A stall (1) as claimed in Claim 5, **characterized in that** a drain (26) is formed in said depression (25) and connected hydraulically to waste water draining means; said depression (25) channelling water to said drain (26).

7. A stall (1) as claimed in Claim 6, **characterized in that** the bottom wall of said depression (25) slopes downwards towards said drain (26).

8. A stall (1) as claimed in at least one of Claims 5 to 7, **characterized in that** a conduit (27) is installed inside said depression (25), is connected to said steam generator (6), and is fitted with said nozzles (7).

9. A stall (1) as claimed in Claim 7, **characterized in that** said conduit (27) is annular and surrounds said hole (24).

10. A stall (1) as claimed in Claim 9, **characterized in that** said nozzles (7) are equally spaced along said conduit (27).

11. A stall (1) as claimed in at least one of the foregoing Claims, **characterized in that** at least one of said lateral walls (4) of the structure (3) is transparent.

12. A stall (1) as claimed in Claim 11, **characterized in that** said lateral walls (4) of said structure (3) are all transparent and made of plastic material or glass.

13. A stall (1) as claimed in at least one of the foregoing Claims, **characterized by** being equipped for chromotherapy.

14. A stall (1) as claimed in any one of the foregoing Claims, **characterized by** being equipped for aromatherapy.

15. A stall (1) as claimed in any one of the foregoing Claims, **characterized by** comprising a device (13) for activating the steam function, and a device (14) for adjusting the temperature of the stall (1).

16. A stall (1) as claimed in any one of the foregoing Claims, **characterized by** comprising a shower head (8); a device (11) for adjusting water supply from said shower head (8); and a mixing device (12) for adjusting the temperature of the water from said shower head (8).

## Patentansprüche

1. Kabine (1) umfassend eine Duschtasse (2), eine durch Seitenwände (4), in denen ein zugang ausgebildet ist, und eine obere Wand (5) definierte Kastenstruktur (3), einen Dampferzeuger' (6) und an der Duschtasse (2) angeordnete Düsen (7) zum Richten von Dampf in die Kabine (1), wobei die Duschtasse einen Grundkörper (18) umfasst, **dadurch gekennzeichnet, dass** die Duschtasse (2) auch eine Trittplatte (21) umfasst, um den Dampf aus den Düsen (7) zu den Seitenwänden (4) umzuleiten, dass zwischen dem Grundkörper (18) und der Trittplatte (21) eine Öffnung (32) in der Nähe der Seitenwände (4) definiert ist und dass die Düsen (7) so unter der Trittplatte (21) installiert sind, dass der Dampf durch die Öffnung (32) steigt.

2. Kabine (1) nach Anspruch 1, **dadurch gekennzeichnet, dass** die Platte (21) entfernbar am Grundkörper (18) montiert ist.

3. Kabine (1) nach Anspruch 2, **dadurch gekennzeichnet, dass** der Grundkörper (18) kastenförmig ist und eine obere Wand (23) mit einem mittleren Durchgangsloch (24) aufweist und dass der Dampferzeuger (6) im Grundkörper (18) untergebracht ist, wobei das Loch (24) den Zugriff auf den vom Grundkörper (18) umschlossenen Raum definiert.

4. Kabine (1) nach Anspruch 3, **dadurch gekennzeichnet, dass** die Platte (21) auf ihrer unteren Fläche einen Vorsprung (31) aufweist, der mit dem Loch (24) in Eingriff steht.

5. Kabine (1) nach Anspruch 3 und/oder 4, **dadurch gekennzeichnet, dass** die obere Wand (23) des Grundkörpers (18) eine Vertiefung (25) aufweist, die das Loch (24) umgibt und in der die Düsen (7) untergebracht sind, wobei die Vertiefung (25) den Dampf aus den Düsen (7) zur Öffnung (32) leitet.

6. Kabine (1) nach Anspruch 5, **dadurch gekennzeichnet, dass** in der Vertiefung (25) ein Abfluss (26) ausgebildet ist, der an Abwasserabflusseinrichtungen hydraulisch angeschlossen ist, wobei die Vertiefung (25) Wasser zum Abfluss (26) leitet.

7. Kabine (1) nach Anspruch 6, **dadurch gekennzeichnet, dass** die Bodenwand der Vertiefung (25) nach unten zum Abfluss (26) abfällt.

8. Kabine (1) nach mindestens einem der Ansprüche 5 bis 7, **dadurch gekennzeichnet, dass** eine Leitung (27) in der Vertiefung (25) installiert, mit dem Dampferzeuger (6) verbunden und mit den Düsen (7) ausgestattet ist.

9. Kabine (1) nach Anspruch 7, **dadurch gekennzeichnet, dass** die Leitung (27) ringförmig ist und das Loch (24) umgibt.

10. Kabine (1) nach Anspruch 9, **dadurch gekennzeichnet, dass** die Düsen (7) gleichmäßig entlang der Leitung (27) beabstandet sind.

11. Kabine (1) nach mindestens einem der vorhergehenden Ansprüche, **dadurch gekennzeichnet, dass** mindestens eine der Seitenwände (4) der Struktur (3) transparent ist.

12. Kabine (1) nach Anspruch 11, **dadurch gekennzeichnet, dass** die Seitenwände (4) der Struktur (3) alle transparent und aus Kunststoffmaterial oder Glas hergestellt sind.

13. Kabine (1) nach mindestens einem der vorhergehenden Ansprüche, **dadurch gekennzeichnet, dass** sie für Farblichttherapie ausgestattet ist.

14. Kabine (1) nach einem der vorhergehenden Ansprüche, **dadurch gekennzeichnet, dass** sie für Aromatherapie ausgestattet ist.

15. Kabine (1) nach einem der vorhergehenden Ansprüche, **dadurch gekennzeichnet, dass** sie eine Vorrichtung (13) zum Anschalten der Dampffunktion und eine Vorrichtung (14) zum Verstellen der Temperatur der Kabine (1) aufweist.

16. Kabine (1) nach einem der vorhergehenden Ansprüche, **dadurch gekennzeichnet, dass** sie einen Duschkopf (8), eine Vorrichtung (11) zum Verstellen der Wasserzufuhr aus dem Duschkopf (8) und eine Mischvorrichtung (12) zum Verstellen der Temperatur des Wassers aus dem Duschkopf (8) umfasst.

## Revendications

1. Cabine (1) comportant un receveur de douche (2) ; une structure de caisson (3) définie par des parois latérales (4), dans lesquelles un accès est formé, et par une paroi supérieure (5) ; un générateur de vapeur (6) ; et des buses (7) situées sur le receveur de douche (2) pour diriger de la vapeur à l'intérieur de la cabine (1) ;
le receveur de douche comportant un corps de base (18) ;
la cabine (1) étant **caractérisée en ce que** ledit receveur de douche (2) comporte aussi une plaque de piétinement (21) pour dévier la vapeur provenant desdites buses (7) en direction desdites parois latérales (4) ; **en ce qu'**entre ledit corps de base (18) et ladite plaque de piétinement (21) est définie une ouverture (32) proche desdites parois latérales (4) ; et **en ce que** lesdites buses (7) sont installées en dessous de ladite plaque de piétinement (21) de sorte que la vapeur monte à travers ladite ouverture (32).

2. Cabine (1) selon la revendication 1, **caractérisée en ce que** la plaque (21) est montée de manière amovible sur ledit corps de base (18).

3. Cabine (1) selon la revendication 2, **caractérisée en ce que** ledit corps de base (18) est en forme de caisson, et comporte une paroi supérieure (23) ayant un trou central traversant (24) ; et **en ce que** ledit générateur de vapeur (6) est reçu à l'intérieur du corps de base (18) ; ledit trou (24) définissant l'accès à l'espace enfermé par ledit corps de base (18).

4. Cabine (1) selon la revendication 3, **caractérisée en ce que** ladite plaque (21) comporte, sur sa face inférieure, une saillie (31) qui vient en prise avec ledit trou (24).

5. Cabine (1) selon la revendication 3 et/ou 4, **caractérisée en ce que** ladite paroi supérieure (23) dudit corps de base (18) comporte un creux (25) entourant ledit trou (24) et recevant lesdites buses (7) ; ledit creux (25) canalisant la vapeur provenant desdites buses (7) vers ladite ouverture (32).

6. Cabine (1) selon la revendication 5, **caractérisée en ce qu'**une évacuation (26) est formée dans ledit creux (25) et connectée hydrauliquement à des moyens d'évacuation d'eau de rejet ; ledit creux (25) canalisant l'eau vers ladite évacuation (26).

7. Cabine (1) selon la revendication 6, **caractérisée en ce que** la paroi inférieure dudit creux (25) s'incline vers le bas en direction de ladite évacuation (26).

8. Cabine (1) selon l'une quelconque des revendications 5 à 7, **caractérisée en ce qu'**un conduit (27) est installé à l'intérieur dudit creux (25), est relié audit générateur de vapeur (6), et est muni desdites buses (7).

9. Cabine (1) selon la revendication 7, **caractérisée en ce que** ledit conduit (27) est annulaire et entoure ledit trou (24).

10. Cabine (1) selon la revendication 9, **caractérisée en ce que** lesdites buses (7) sont espacées de manière égale le long dudit conduit (27).

11. Cabine (1) selon l'une quelconque des revendications précédentes, **caractérisée en ce qu'**au moins une desdites parois latérales (4) de la structure (3) est transparente.

12. Cabine (1) selon la revendication 11, **caractérisée en ce que** lesdites parois latérales (4) de ladite structure (3) sont toutes transparentes et constituées d'une matière plastique ou de verre.

13. Cabine (1) selon l'une quelconque des revendications précédentes, **caractérisée en ce qu'**elle est équipée pour une chromothérapie.

14. Cabine (1) selon l'une quelconque des revendications précédentes, **caractérisée en ce qu'**elle est équipée pour une aromathérapie.

15. Cabine (1) selon l'une quelconque des revendications précédentes, **caractérisée en ce qu'**elle comporte un dispositif (13) pour activer la fonction vapeur, et un dispositif (14) pour ajuster la température de la cabine (1).

16. Cabine (1) selon l'une quelconque des revendications précédentes, **caractérisée en ce qu'**elle comporte une pomme de douche (8) ; un dispositif (11) pour ajuster l'alimentation en eau à partir de ladite pomme de douche (8) ; et un mélangeur (12) pour ajuster la température de l'eau provenant de ladite pomme de douche (8).
